# DEMANDE DE BREVET EUROPEEN

(11) **EP 2 005 882 A1**
(43) Date de publication de la demande: **24.12.2008**
(21) Numéro de dépôt: 08290544.9
(22) Date de dépôt: 12.06.2008
(51) Int. Cl.: A61B 5/00, A61M 39/10

(54) **Dispositif de raccordement fluidique, système et méthode de prélèvement en continu de microéchantillons fluides utilisant ce dispositif**

(30) Priorité: 19.06.2007 FR 0704349
(71) Demandeur: COMMISSARIAT A L'ENERGIE ATOMIQUE, 75015 Paris (FR)
(72) Inventeur: Reymond, Jean-Marc, 78470 Saint Rémy-Lès-Chevreuse (FR); Kerhoas-Cavata, Sophie, 78125 Raizeux (FR); Mangeot, Philippe, 94270 Le Kremlin-Bicêtre (FR); Boisgard, Raphael, 91620 Nozay (FR)
(74) Mandataire: Bolinches, Michel Jean-Marie

(57) **Abrégé**

La présente invention concerne un dispositif de raccordement fluidique et un système et une méthode de prélèvement automatisé et en continu de microéchantillons fluides utilisant ce dispositif.

Un dispositif (20) selon l'invention, qui est destiné à être raccordé à un premier conduit (21) par une première ouverture et qui comporte une seconde ouverture traversée par un second conduit (28), comporte :
- un raccord fluidique femelle (22) qui définit la première ouverture et qui comprend une surface interne d'emmanchement (23) se terminant par une extrémité femelle (24) où débouche ce premier conduit, et
- un raccord fluidique mâle (25) qui définit la seconde ouverture en étant emmanché dans le raccord femelle via sa surface externe (26) et qui se termine par une extrémité radiale mâle (27) à l'intérieur du raccord femelle.

Selon l'invention, ce second conduit est formé d'un micro-tube souple enfoncé dans le raccord mâle au-delà de l'extrémité mâle, l'extrémité libre du second conduit appuyant de manière étanche contre l'extrémité femelle de sorte à minimiser le volume mort entre le premier conduit et le raccord mâle.

## Description

La présente invention concerne un dispositif de raccordement fluidique exempt de volume mort, qui est destiné à transférer un fluide, ainsi qu'un système et une méthode de prélèvement automatisé et en continu d'une série de microéchantillons liquides de telle sorte que les échantillons ainsi prélevés soient discrétisés dans l'espace et dans le temps jusqu'à leur stockage temporaire, pour traitement ultérieur. L'invention s'applique plus particulièrement, mais non exclusivement, à un dispositif de raccordement fluidique pour le transfert de microéchantillons de sang total d'un mammifère, par exemple un rat ou une souris.

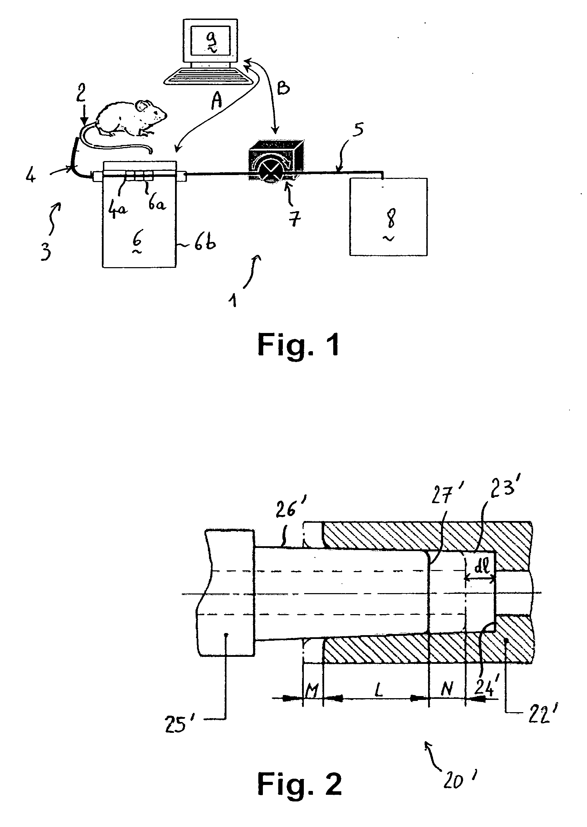

Les dispositifs de raccordement fluidiques qui sont utilisés en médecine et en biologie en relation avec un cathéter ou similaire, d'une part, et avec un micro-tube souple, d'autre part, sont usuellement des raccords standardisés de type « Luer » définis par la norme ISO 59461 de 1986, ou bien de type « Luer Lock » définis par la norme ISO 594-2 de 1998. En référence à la figure 2 annexée à la présente description, les dispositifs de raccordement usuels 20' comportent essentiellement :
- un raccord fluidique femelle 22' dans lequel est enfoncé un cathéter et qui comprend une surface interne d'emmanchement conique 23' convergeant vers une extrémité femelle 24' où débouche ce cathéter, et
- un raccord fluidique mâle 25' qui est emmanché dans le raccord femelle 22' via sa surface externe conique 26' avec la même conicité que la surface interne 23' du raccord femelle, et qui converge vers une extrémité mâle 27' en laissant à l'état d'enfoncement maximal une distance dl avec l'extrémité femelle 24' (voir figure 2).

Le tableau 1 ci-après recense les principales caractéristiques dimensionnelles des dispositifs de raccordement de type « Luer » (angle de conicité de 6 %) en fonction du matériau utilisé, selon la norme précitée.

**Tableau 1 :**

| Cote | | Désignation | Dimensions (mm) | |
|---|---|---|---|---|
| | | | Matériau rigide | Matériau semi-rigide |
| Dimensions de base | dₘᵢₙ | Diamètre minimal de l'extrémité du raccord conique mâle (diamètre de référence) | 3,925 | 3,925 |
| | dₘₐₓ | Diamètre maximal de l'extrémité du raccord conique mâle | 3,990 | 4,027 |
| | Dₘᵢₙ | Diamètre minimal de l'ouverture du raccord conique femelle | 4,270 | 4,270 |
| | Dₘₐₓ | Diamètre maximal de l'ouverture du raccord conique femelle | 4,315 | 4,315 |
| | E | Longueur minimale du raccord conique mâle | 7,500 | 7,500 |
| | F | Profondeur minimale du raccord conique femelle | 7,500 | 7,500 |
| Autres dimensions | L* | Pénétration minimale | 4,665 | 4,050 |
| | M* | Ecart sur la pénétration du raccord femelle | 0,750 | 0,750 |
| | N* | Ecart sur la pénétration du raccord mâle | 1,083 | 1,700 |
| | R**ₘₐₓ | Rayon de courbure | 0,5 | 0,5 |

| | | | | |
|---|---|---|---|---|
| En référence aux symboles * et ** de ce tableau : * les dimensions L, M, N résultent des dimensions de base, et ** ou chanfrein d'entrée équivalent n'ayant pas d'angles vifs | | | | |

Un inconvénient majeur des dispositifs de raccordement définis par les normes précitées est que la distance dl (typiquement de 3 mm environ) entre les extrémités respectives des raccords mâle et femelle génère un volume mort intrinsèque qui devient pénalisant dans diverses circonstances. La plus fréquente se présente lorsque les raccords servent à la circulation de très petits échantillons liquides, d'un ordre de grandeur comparable ou même inférieur à ce volume mort. A titre d'exemple, lors de l'injection ou du prélèvement de microéchantillons de sang de mammifère, on utilise généralement un conduit souple muni d'un raccord d'un diamètre d'environ 4 mm, l'espace ainsi délimité déterminant un volume mort de l'ordre de 15 µL à 30 µL, lequel peut engendrer les problèmes suivants en utilisation :
- plusieurs microéchantillons successifs servant à remplir ce volume mort, cela retarde la traversée des premiers microéchantillons et implique de prélever un volume de fluide plus grand qui est perdu, et
- la section du conduit fluidique s'élargissant considérablement du fait de ce volume mort, différents microéchantillons s'y mélangent les uns aux autres, ce qui est pénalisant si le fluide est un liquide, et que l'on exploite ultérieurement ces microéchantillons par exemple à des fins d'analyse. Cela nuit à leur traçabilité et est particulièrement préjudiciable dans le cas du suivi de phénomènes biologiques rapides au cours desquels il est fondamental que chaque microéchantillon puisse conserver ses caractéristiques initiales tout au long de la ligne de distribution.

D'autres inconvénients peuvent aussi en résulter, comme une nécessité de purger la canalisation si l'on veut éviter tout mélange avec un fluide résiduel, cette opération de purge étant d'ailleurs rendue particulièrement délicate par l'existence et la forme du volume mort.

Il est par ailleurs connu par le document US-A-4 966 588 d'utiliser, pour l'injection d'une substance liquide thérapeutique, un dispositif de raccordement fluidique comportant essentiellement :
- un raccord mâle emmanché dans un raccord femelle via des surfaces respectives d'emmanchement cylindriques munies d'épaulements, ce raccord femelle étant destiné à recevoir une canule formant embout d'injection, et
- une aiguille rigide d'injection qui est insérée de manière traversante à la fois dans le raccord mâle, le raccord femelle et la canule en venant percer une rondelle d'étanchéité positionnée entre les deux raccords, et qui est destinée à être implantée dans le corps à traiter.

Un inconvénient majeur de ce dispositif à aiguille d'injection rigide qui traverse de part en part les raccords et la canule réside dans le faible diamètre de cette aiguille, en comparaison de celui de l'ouverture du raccord mâle. Un autre inconvénient tient au fait que la rondelle d'étanchéité peut être déplacée involontairement suite au retrait de l'aiguille des raccords après injection, ce qui peut occasionner des problèmes de positionnement en utilisation multiple, ou bien imposer un changement à chaque utilisation de la rondelle d'étanchéité, peu accessible.

Enfin, un autre inconvénient réside dans le fait que l'aiguille qui perfore la rondelle d'étanchéité a nécessairement une certaine longueur, qui peut devenir gênante en amont ou en aval de l'aiguille. Ainsi, elle n'est pas du tout adaptée à l'injection d'un liquide ou au prélèvement d'échantillons de sang dans un petit mammifère, tel qu'un rat ou une souris. Chez un rat en particulier, on utilise généralement la veine (ou pour une injection, l'artère) caudale de l'animal, en introduisant un cathéter directement relié au conduit souple par un raccord. L'introduction d'une aiguille métallique d'une certaine longueur à travers la rondelle d'étanchéité suppose que cette aiguille dépasse à l'intérieur de la veine (ou de l'artère), qui se trouverait alors blessée lors des mouvements de la queue de l'animal.

Un but de la présente invention est de proposer un dispositif de raccordement fluidique destiné à transférer un fluide, tel que des microéchantillons à prélever (dans ce premier cas, par exemple du sang) ou à injecter (dans ce second cas, un liquide de manière générale), et destiné à être raccordé à un premier conduit par une première ouverture du dispositif, lequel comporte une seconde ouverture traversée par un second conduit destiné à communiquer avec le premier conduit pour transférer ce fluide, le dispositif comportant :
- un raccord fluidique femelle qui définit ladite première ouverture dans laquelle est destiné à être enfoncé ledit premier conduit et qui comprend une surface interne d'emmanchement se terminant par une extrémité radiale femelle où débouche ce premier conduit, et
- un raccord fluidique mâle qui définit ladite seconde ouverture en étant emmanché dans le raccord femelle via sa surface externe et qui se termine par une extrémité radiale mâle à l'intérieur du raccord femelle,
dispositif qui remédie à l'ensemble des inconvénients précités.

A cet effet, un dispositif selon l'invention est tel que ce second conduit est formé d'un micro-tube souple enfoncé dans le raccord mâle axialement au-delà de ladite extrémité mâle, l'extrémité libre de ce second conduit appuyant de manière étanche contre ladite extrémité femelle de sorte à minimiser le volume mort entre le premier conduit et le raccord mâle.

Par « microéchantillons », on entendra dans la présente description des échantillons liquides, tels que des échantillons sanguins, présentant chacun un volume inférieur à 100 µL et, de préférence, inférieur ou égal à 30 µL (i.e. typiquement des échantillons prélevés sur des petits animaux). De préférence, chaque microéchantillon selon l'invention est un échantillon de sang total d'un mammifère de type rat ou souris et présente un volume allant de 8 µL à 30 µL.

On notera que ce volume mort minimisé par ce dispositif selon l'invention permet de remédier aux inconvénients précités liés à la circulation de microéchantillons - typiquement de volumes inférieurs ou égaux à 30 µL - avec les dispositifs de raccordement connus (traversée retardée des premiers microéchantillons, pertes de volumes liquides prélevés et mélanges des microéchantillons nuisant à leur traçabilité, notamment).

Selon une autre caractéristique de l'invention, ladite surface interne d'emmanchement du raccord femelle peut être une surface conique convergeant vers ladite extrémité femelle avec une même conicité que ladite surface externe du raccord mâle qui converge vers ladite extrémité mâle. De préférence, lesdits raccords mâle et femelle peuvent être alors tous deux des raccords de type « Luer », tels que définis par la norme ISO 59461 de 1986, ou bien de type « Luer Lock », tels que définis par la norme ISO 594-2 de 1998.

De préférence, ledit second conduit peut dépasser de ladite extrémité du raccord mâle d'une longueur axiale qui est au moins égale à la distance minimale dl séparant lesdites extrémités respectives des deux raccords lorsque le raccord mâle est enfoncé en position de connexion dans le raccord femelle, conformément à l'une ou à l'autre desdites normes.

Egalement à titre préférentiel, ledit second conduit est pourvu, autour de sa paroi cylindrique et à proximité de son extrémité libre, d'un moyen de rigidification apte à le rigidifier à l'intérieur dudit raccord femelle.

Avantageusement, ledit moyen de rigidification est formé d'une bague réalisée dans un matériau qui présente une rigidité au moins égale et préférentiellement supérieure à celle dudit second conduit, et qui est apte à être solidarisé avec lui, cette solidarisation pouvant être directe ou par l'intermédiaire dudit raccord fluidique mâle. Dans ce dernier cas, le raccord fluidique mâle est nécessairement solidaire dudit second conduit.

Selon une variante préférentielle, le moyen de rigidification est solidaire de l'extrémité du second conduit qu'il enserre, et il est monté en appui entre ce dernier et ladite surface interne d'emmanchement conique du raccord femelle. A titre d'exemple, ce moyen de rigidification peut être réalisé à base d'une résine polymérique.

Cette bague peut être rapportée autour du second conduit ou bien coextrudée avec ce dernier. Selon une variante plus adaptée à la production industrielle, le raccord mâle peut être réalisé en une seule opération par moulage, après un léger ré-usinage de la forme initiale du moule permettant l'incorporation de ce moyen de rigidification.

Quant audit premier conduit équipant le dispositif de raccordement selon l'invention, il s'agit avantageusement d'un micro-tube souple adapté pour le prélèvement ou l'injection dudit liquide dans un animal, tel qu'un cathéter souple à implanter dans la veine caudale d'un petit mammifère en vue du prélèvement de microéchantillons sanguins.

Un système automatisé selon l'invention de prélèvement en continu d'une série de microéchantillons liquides d'un corps contenant un liquide à prélever, tels que des microéchantillons sanguins d'un mammifère de type rat ou souris, le système comportant une succession de conduits qui sont reliés entre eux par une pompe doseuse, telle qu'une pompe péristaltique, pour l'aspiration par à-coups d'une quantité déterminée du liquide à acheminer vers un conteneur de stockage, cette succession de conduits comportant un premier conduit souple de type cathéter qui est destiné à être implanté dans ce corps et qui est raccordé à un dispositif de raccordement pour le transfert des microéchantillons vers la pompe, est caractérisé en ce que ce dispositif de raccordement est tel que défini ci-dessus (i.e. en incluant ledit second conduit prolongeant ce premier conduit).

Selon une autre caractéristique de l'invention, ledit système de prélèvement est relié à un dispositif de commande assisté par ordinateur, qui est notamment prévu pour commander ladite pompe.

Selon une autre caractéristique de l'invention, ladite succession de conduits présente une section transversale sensiblement constante, de sorte que les microéchantillons s'y succédant circulent chacun sur une longueur axiale qui est supérieure à au moins cinq fois la plus grande dimension transversale intérieure de ces conduits (e.g. leur diamètre interne).

Si l'invention est destinée à produire une succession discontinue d'échantillons en vue d'alimenter un dispositif aval, la succession de conduits peut se terminer avantageusement par un tronçon plus rigide faisant office de microbuse. Dans ce cas, afin de faciliter le dépôt du microéchantillon, l'extrémité de la microbuse vient en contact avec la paroi réceptrice dudit dispositif aval, cette paroi faisant de préférence un angle compris entre 5° et 85° avec le plan de section de l'extrémité de ladite microbuse. Préférentiellement, cet angle est sensiblement égal à 45°. En effet, si cet angle était supérieur à 90°, alors tout ou partie de chaque goutte formée par la microbuse resterait accrochée à celle-ci pour des raisons de tension superficielle.

Selon une autre caractéristique essentielle de l'invention, les élargissements de section transversale de ladite succession de conduits sont tous inférieurs ou égaux à 20 % en termes de ratios de surfaces, de telle sorte que les microéchantillons se succédant dans ces conduits soient discrétisés dans l'espace et dans le temps, en particulier pour que ceux dont le volume est inférieur ou égal à 30 µL, ne s'y mélangent pratiquement pas entre eux.

Selon une autre caractéristique de l'invention, ledit micro-tube souple formant ledit second conduit se prolonge par au moins un tronçon qui est au moins partiellement aplati et de section transversale oblongue, et qui est adapté pour minimiser l'atténuation de particules émises par chaque microéchantillon liquide circulant dans ce tronçon de sorte à optimiser le comptage de ces particules, telles que des électrons ou des positons issus de la désintégration bêta moins ou bêta plus, ou encore des photons par exemple dans le cas d'un comptage par fluorescence.

De préférence, ledit tronçon aplati présente une section transversale sensiblement rectangulaire dont les grands côtés et/ou les petits côtés sont incurvés selon des courbures symétriques l'une de l'autre, de sorte que ce tronçon présente au moins en partie une face externe sensiblement convexe ou concave.

Avantageusement, ledit tronçon aplati peut présenter un ratio [hauteur interne / largeur interne] inférieur à 20 % et de préférence compris entre 5 % et 10 %, où la hauteur et la largeur internes représentent respectivement la plus petite et la plus grande des dimensions transversales de ce tronçon, mesurées selon deux directions sensiblement perpendiculaires.

Egalement avantageusement, le ratio surfacique de la section de passage dudit tronçon aplati sur celle de chaque portion cylindrique adjacente à ce tronçon peut être inférieur ou égal à 35 %.

A titre préférentiel, ladite hauteur interne du tronçon aplati peut être inférieure à 500 µm et de préférence comprise entre 100 µm et 200 µm, la ou chaque portion cylindrique adjacente pouvant présenter un diamètre de l'ordre de 1 mm.

Selon une autre caractéristique de l'invention, ledit tronçon aplati présente avantageusement une paroi d'épaisseur e (exprimée en µm) et de masse volumique d (exprimée en g/cm3) dont le produit e.d est inférieur à 100 et de préférence inférieur à 50, de telle sorte que l'atténuation par ce tronçon des particules à compter soit minimisée lorsque ces particules sont des électrons ou des positons (l'atténuation étant de manière connue dans ces deux cas proportionnelle à ce produit e.d).

Avantageusement, ledit tronçon aplati est à base d'un polyimide, par exemple de dénomination « Kapton » et de masse volumique comprise entre 1,3 et 1,5 g/cm3, et ce tronçon aplati présente une paroi d'épaisseur inférieure à 50 µm et de préférence inférieure à 30 µm. Ce tronçon aplati est par exemple obtenu par thermoformage.

Selon une autre caractéristique de l'invention, ledit tronçon aplati est équipé, en regard de ses grandes faces et venant en dépassement de ses petites faces, de deux ensembles de détecteurs aptes à compter lesdites particules de chaque microéchantillon liquide y circulant.

On notera que ce dépassement des détecteurs permet d'optimiser la « capture » des particules à compter (notion géométrique d'acceptance).

Avantageusement, lesdits détecteurs sont agencés contre ou à proximité immédiate desdites grandes faces.

Selon une autre caractéristique de l'invention, les microéchantillons prélevés sont du sang total de mammifère comprenant un plasma et des globules, et ledit système peut comporter, en amont de ladite pompe doseuse qui est pilotée par ordinateur :
- un dispositif de comptage de radioactivité α, β ou γ (particules α, γ, électrons ou positons) fortement atténuée par les matériaux qu'elle traverse, émise par un radiotraceur dilué dans ce sang total, ce dispositif de comptage étant également piloté par ordinateur et comprenant à l'intérieur d'un boîtier de mesure lesdits ensembles de détecteurs, tels que des diodes au silicium, qui sont répartis sensiblement contre lesdites deux plaques, ainsi qu'une carte électronique de traitement et d'interfaçage de ces mesures, et
- un module électronique pour la lecture de ces détecteurs, l'acquisition et le transfert de données.

On notera que ce système peut ainsi avantageusement réaliser ces mesures immédiatement après les prélèvements des microéchantillons.

Une méthode automatisée selon l'invention de prélèvement en continu de microéchantillons liquides d'un corps contenant le liquide à prélever, tels que des microéchantillons sanguins d'un mammifère de type rat ou souris, est caractérisée en ce que l'on prélève en continu, par un système automatisé de prélèvement tel que défini ci-dessus, ces microéchantillons selon une fonction temporelle monotone via l'envoi à ce système de prélèvement et à des instants tᵢ préprogrammés, par un dispositif de commande assisté par ordinateur, de signaux de prélèvement d'un microéchantillon de volume préprogrammé, pour que ces microéchantillons ainsi prélevés via la pompe doseuse se succèdent spatialement et temporellement dans ladite succession de conduits, jusqu'audit conteneur de stockage.

Avantageusement, on notera que la durée d'échantillonnage minimale est d'une seconde avec la méthode de prélèvement en continu selon l'invention, ce qui représente la moitié de la durée d'échantillonnage utilisée dans l'état de l'art.

Ainsi, le système de prélèvement selon l'invention peut permettre notamment de réaliser et d'enchaîner automatiquement (i.e. sans aucune intervention manuelle) une séquence de deux fonctions toutes deux automatisées consistant, d'une part, en un prélèvement de microéchantillons - de préférence sanguins - discrétisés temporellement et spatialement (aisément séparables par exemple via un bris du micro-tube) et, d'autre part, par exemple en une séparation spatiale dans chaque microéchantillon de l'une au moins de ses phases pour la soumettre à une action différentiée, notamment en vue de la mesure de la fonction d'entrée pour l'imagerie nucléaire sur des mammifères de petite taille, en particulier pour l'imagerie quantitative de traceurs en tomographie par émission de positons (TEP),

On notera que la petite taille des animaux qui sont de préférence utilisés pour le prélèvement des microéchantillons requiert de limiter le volume total de ces microéchantillons à une quantité qui est compatible non seulement avec la santé de l'animal, mais encore avec une perturbation aussi faible que possible de son métabolisme.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec les dessins joints, parmi lesquels :
- la figure 1 est une vue schématique partielle d'un système automatisé de prélèvement selon l'invention incluant un système de mesure, tel qu'un compteur de particules issues de la radioactivité bêta, agencé en amont d'un dispositif d'échantillonnage des microéchantillons prélevés,
- la figure 2 est une vue partielle en coupe radiale d'un dispositif de raccordement à raccords mâle et femelle de type connu qui est destiné à être connecté, d'une part, à un cathéter et, d'autre part, à un micro-tube souple pour le prélèvement des microéchantillons,
- la figure 3 est une vue partielle en coupe radiale d'un dispositif de raccordement à raccords mâle et femelle selon l'invention qui est destiné à être connecté à ce cathéter et qui forme une partie amont du système de prélèvement de la figure 1,
- la figure 4 est une photographie illustrant la forme et les dimensions relatives, en comparaison d'une pièce de 10 centimes d'euro, d'un conduit du système de prélèvement selon l'invention prévu en aval de ce dispositif de raccordement et présentant un tronçon aplati pour le comptage de particules réalisé par le système de mesure de la figure 1,
- la figure 4a est une vue schématique en section transversale de ce tronçon aplati selon l'invention équipé des deux ensembles de détecteurs illustrés à la figure 1, et
- la figure 5 est un graphique illustrant l'efficacité de détection en radiotraceur ¹⁸F en fonction du seuil de détection, pour trois types de conduits de prélèvement comprenant ce tronçon aplati de mesure selon l'invention et, à titre d'essais comparatifs, deux micro-tubes cylindriques.

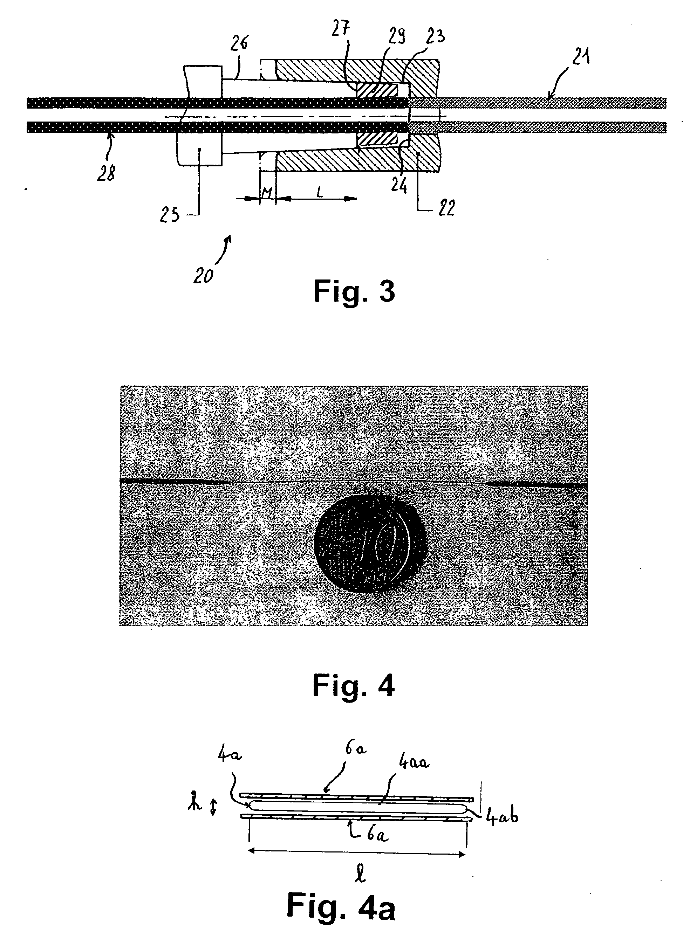

La figure 1 illustre à titre d'exemple une installation 1 automatisée et en continu pour procéder successivement et en continu au prélèvement, au stockage temporaire et à des mesures de microéchantillons sanguins d'un petit mammifère 2 par exemple de type ras ou souris, au moyen d'un système de prélèvement 3 incluant une succession de conduits 4 et 5 de type micro-tubes ou capillaires souples. Ce système de prélèvement 3 comporte essentiellement :
- un cathéter 21 qui est équipé d'un dispositif de raccordement 20 (tous deux visibles à la figure 3) et qui est destiné à aspirer par à-coups une même quantité de sang à prélever, via une pompe péristaltique 7,
- un système de comptage 6 de particules présentes dans les microéchantillons prélevés, qui est dans cet exemple un compteur de particules issues de la radioactivité bêta 6 pour des microéchantillons de sang total et qui est placé au plus près du point de prélèvement en étant quasiment au contact d'un tronçon de mesure 4a de cette succession de conduits 4, 5 (comme développé ci-après, ce tronçon 4a présente des caractéristiques de forme et de matériau optimisées pour ce comptage et est centré par rapport aux diodes de détection 6a que comporte le compteur 6),
- un système d'échantillonnage 8 agencé en aval de la pompe péristaltique 7, où ces microéchantillons prélevés et analysés sont stockés et traités, et
- un dispositif de commande 9 assisté par ordinateur pour la commande de l'ensemble du système 3, en incluant cette pompe 7 (voir les doubles flèches A et B à la figure 1 pour cette commande).

Selon l'invention, la succession de conduits 4 et 5 est telle que les élargissements de section transversale présents sur ces conduits sont tous inférieurs ou égaux à 20 % en termes de ratios de surfaces, de sorte que les microéchantillons se suivant dans cette succession de conduits 4 et 5 ne s'y mélangent pratiquement pas entre eux par diffusion. De cette manière, ces microéchantillons sont discrétisés dans l'espace et dans le temps

Comme illustré à la figure 3, le dispositif de raccordement 20 selon l'invention comporte, connecté au cathéter souple 21 qui est destiné à être implanté par exemple dans la veine caudale du mammifère 2 et qui présente par exemple un diamètre extérieur de 1,5 mm et un diamètre intérieur de 0,8 mm :
- un raccord fluidique femelle 22 dans lequel est enfoncé le cathéter 21 et qui comprend une surface interne d'emmanchement conique 23 convergeant vers une extrémité radiale femelle 24 où débouche le cathéter 21,
- un raccord fluidique mâle 25 qui est emmanché dans le raccord femelle 22 via sa surface externe conique 26 avec la même conicité que cette surface interne 23 du raccord femelle 22, et qui converge vers une extrémité radiale mâle 27, et
- un micro-tube souple 28 en PEBD (polyéthylène de basse densité, tel qu'un « PEBD 50 » ou un « PEBD 100 ») qui est adapté pour acheminer vers un conteneur de stockage les microéchantillons prélevés en étant discrétisés dans l'espace et dans le temps, et qui est enfoncé dans le raccord mâle 25 axialement au-delà de l'extrémité mâle 27 et de manière immédiatement adjacente à l'extrémité femelle 24 en regard, de sorte à minimiser le volume mort localisé à l'intérieur du raccord femelle 22 entre ces deux extrémités respectives 27 et 24.

Plus précisément, ces raccords 22 et 25 sont des raccords de type « Luer », tels que définis par la norme ISO 59461 de 1986, ou bien de type « Luer Lock », tels que définis par la norme ISO 594-2 de 1998.

De préférence, le micro-tube 28 dépasse de l'extrémité mâle 27 d'une longueur axiale qui est au moins égale à la distance minimale dl séparant les extrémités 27 et 24 lorsque le raccord mâle 25 est enfoncé en position de connexion dans le raccord femelle 22, conformément à l'une ou à l'autre de ces normes. En fait et comme illustré à la figure 3, ce micro-tube 28 appuie de manière étanche contre l'extrémité femelle 24.

Le raccord mâle 25 selon l'invention permet de s'affranchir vu volume mort inhérent à la différence de longueur dl précitée en référence aux dispositifs de raccordement connus illustrés à la figure 2.

Comme illustré à cette figure 3, le micro-tube 28 de l'invention est pourvu, à son extrémité libre adjacente au cathéter 21, d'un anneau de rigidification 29 monté entre la paroi cylindrique du micro-tube 28 et la surface interne 23 du raccord femelle 22. Avantageusement, le moyen de rigidification 29 est formé d'une bague réalisée dans un matériau de rigidité au moins égale et préférentiellement supérieure à celle du micro-tube 28, et apte à se solidariser avec lui, cette solidarisation pouvant être directe ou par l'intermédiaire du raccord mâle 25. Dans ce dernier cas, le raccord mâle 25 est nécessairement solidaire du micro-tube 28.

Selon une variante préférentielle, la bague 29 est solidaire de l'extrémité du micro-tube 28 et peut être réalisée à base d'une résine polymérique. Cette bague 29 peut être rapportée autour du micro-tube 28 ou bien coextrudée avec ce dernier.

On a fabriqué le dispositif de raccordement 20 selon l'invention à partir d'un dispositif 20' de l'art antérieur, en y perçant un orifice de section annulaire pour y introduire à force le micro-tube 28 (la coupe franche de l'extrémité étant perpendiculaire à l'axe du micro-tube 28).

On notera que le raccord mâle 25 selon l'invention est ainsi compatible avec toute la gamme des matériels standards « Luer » femelle selon les normes précitées, et qu'il permet avantageusement de supprimer le volume mort dans le dispositif de raccordement 20 (ce volume mort pouvant correspondre à une augmentation de section transversale de 20 % par rapport à la section minimale du conduit) et, par conséquent, de transférer notamment des microéchantillons sanguins sans risque de mélange de ceux-ci (avec donc une traçabilité parfaite, les échantillons en sortie de ligne étant conformes à ceux en entrée de ligne). En outre, cela permet de ne pas devoir attendre le prélèvement de trop nombreux microéchantillons pour commencer les mesures ou analyses, et également de ne pas gaspiller un volume de liquide correspondant au volume mort.

Comme illustré aux figures 4 et 4a, on a prévu dans la succession de conduits de prélèvement selon l'invention un tronçon aplati de mesure 4a qui prolonge ledit second conduit 28 de la figure 3 et qui est conçu pour optimiser le comptage par le système de comptage 6 de particules de la figure 1 (tel que le compteur bêta, que l'on utilise avantageusement pour la mesure de la fonction d'entrée du petit mammifère 2). On a ainsi choisi de réduire le volume de détection et d'augmenter l'efficacité de comptage.

A cet effet, on a façonné par thermoformage ce tronçon aplati de mesure 4a de section transversale oblongue qui est réalisé en un polyimide de dénomination « Kapton » (de masse volumique égale à 1,42 g/cm³, avec une paroi de 25 µm ±10 % d'épaisseur) et qui relie entre eux deux conduits cylindriques par exemple en PEBD de diamètre interne par exemple égal à 1 mm environ. Comme illustré à la figure 4a, les diodes de détection 6a précitées du système 6 de comptage sont agencées de part et d'autre de ce tronçon 4a par rapport à sa plus petite dimension transversale constituée ici par sa hauteur h.

Dans cet exemple de réalisation, le tronçon aplati 4a présente une section transversale sensiblement rectangulaire dont les petits côtés sont incurvés selon des courbures convexes symétriques l'une de l'autre, et ce tronçon présente un ratio [hauteur interne h / largeur interne l] d'environ 8 %, où la hauteur et la largeur internes sont respectivement égales à 130 µm et à 1490 µm.

Quant au ratio surfacique de la section de passage du tronçon aplati 4a - d'environ 0,19 mm² - sur celle de chaque portion cylindrique adjacente (de section interne d'environ 0,78 mm²), il est légèrement inférieur à 25 %.

En outre, le tronçon aplati 4a présente une paroi d'épaisseur e et de masse volumique d dont le produit e.d est sensiblement égal à 35,5 (avec e = 25 µm et d = 1,42 g/cm³), ce qui est très nettement inférieur aux valeurs usuellement utilisées qui sont généralement comprises entre 150 et 200 pour les micro-conduits réalisés en PEBD (qui présentent une densité inférieure à celle du « Kapton » mais une épaisseur nettement supérieure), de telle sorte que l'atténuation par ce tronçon 4a selon l'invention des particules à compter, telles que des électrons ou des positons, est nettement minimisée.

Comme illustré à la figure 4a, le tronçon aplati 4a est équipé, en regard de ses grandes faces et venant en dépassement de ses petites faces, de deux ensembles desdites diodes de détection 6a aptes à compter lesdites particules de chaque microéchantillon liquide y circulant (ce dépassement des diodes 6a permet d'optimiser la « capture » des particules à compter).

Le procédé de thermoformage utilisé pour l'obtention de ce tronçon aplati 4a selon l'invention comprend notamment les étapes suivantes :
- mise en place du tronçon 4a à froid dans le gabarit de formage,
- raccordement de ses deux extrémités à des micro-tubes souples pour la mise en pression,
- mise sous pression (1,5 bars de pression relative),
- chauffage du gabarit à 300° C pendant 15 minutes,
- refroidissement sous pression, et
- chute lente de la pression après refroidissement.

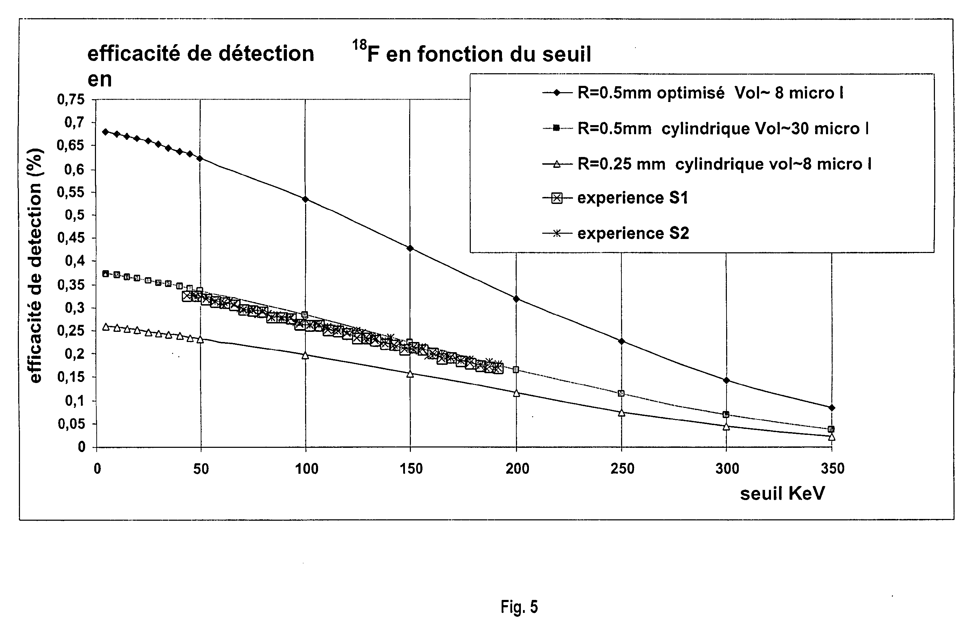

Le graphique de la figure 5 illustre, sous forme de courbes de simulation confirmées par l'expérience, les résultats d'efficacité de comptage obtenus pour deux séries d'expériences S1 et S2, réalisées chacune :
- avec un conduit selon l'invention de rayon égal à 0,5 mm pour les portions cylindriques et incorporant ce tronçon 4a aplati, avec un volume de microéchantillon sanguin prélevé de 8 µL (compatible avec une souris),
- avec un premier conduit « témoin » cylindrique sur toute sa longueur (i.e. sans tronçon aplati) présentant un rayon de 0,5 mm, avec un volume de microéchantillon sanguin prélevé de 30 µL (compatible avec un rat), et
- avec un second conduit « témoin » cylindrique sur toute sa longueur (i.e. sans tronçon aplati) présentant un rayon de 0,25 mm, avec un volume de microéchantillon sanguin prélevé de 8 µL.

Grâce à ce tronçon aplati 4a, on note que l'efficacité de détection des positons augmente, passant de 32 % avec les micro-tubes cylindriques à plus de 60 % avec le micro-tube de l'invention, pour le seuil minimum (d'environ 46 Kev). Le gain est même plus important car un conduit micro-tubulaire cylindrique compatible avec 8 µL donnerait environ 25 % d'efficacité. Le micro-tube optimisé selon l'invention permet ainsi de travailler avec des échantillons de 8 µL, en atteignant plus de 60 % d'efficacité au seuil minimum, contre seulement 25 % avec un micro-tube entièrement cylindrique sur sa longueur, ce qui rend le système de prélèvement 3 selon l'invention particulièrement bien adapté à la mesure de la fonction d'entrée d'une souris.

En relation avec la figure 1, on va à présent décrire plus précisément le système et la méthode de comptage des rayonnements bêta utilisés en relation avec le micro-tube 28 inséré dans le dispositif de raccordement 20 de la figure 3 et se prolongeant par le tronçon aplati 4a du conduit de prélèvement selon l'invention illustré à la figure 4.

Quelques centimètres en aval de ce dispositif de raccordement 20, chaque microéchantillon passe au plus près du compteur de particules issues de la radioactivité bêta 6, pour lequel l'épaisseur de la paroi du conduit n'apporte qu'une très faible atténuation. Le tronçon aplati 4a, fixé dans le boîtier 6b du compteur 6, permet de minimiser le taux d'annihilation des positons dans les parois, et sa géométrie est telle qu'il peut contenir le volume d'un échantillon (de 30 µL ou de 8 µL) correctement centré sous les six diodes de détection en silicium (10 x 10 x 0,3 mm³) entourant le tronçon 4a, comme illustré à la figure 1. Ces diodes 6a sont elles-mêmes entourées d'un blindage de plomb de 2 cm d'épaisseur destiné à supprimer le bruit physique venant des photons issus de l'animal 2. Le reste de ce système de mesure 6 comporte une carte électronique de traitement et interfaçage, l'ensemble privilégiant la compacité et la robustesse en formant un boîtier 6b de petites dimensions (16 x 11 x 4 cm³).

Il est avantageux, pour minimiser la probabilité d'annihilation dans le sang d'un positon issu de la radioactivité béta, de donner au micro-tube souple la forme aplatie du tronçon 4a, au moins à l'endroit de son passage devant les diodes 6a. Par ailleurs, comme expliqué précédemment, cette configuration géométrique assure un étalement du liquide en une nappe fine, ce qui augmente la surface de liquide en regard des surfaces détectrices. La configuration retenue pour le système de mesure 6 est la suivante.

Le tronçon aplati 4a, de 25 µm d'épaisseur de paroi, est placé en « sandwich » entre les diodes 6a de 0,3 mm d'épaisseur (trois diodes 6a en haut et trois autres en bas).

L'électronique de lecture de ces diodes 6a ainsi que l'électronique gérant l'acquisition et le transfert de données ont été intégrées dans un unique module électronique, qui a été optimisé afin de réduire au maximum le bruit électronique permettant de minimiser le seuil de détection pour une meilleure efficacité.

L'électronique « front-end » (mise en forme et discriminateur) est assurée par un « ASIC » (comprenant 16 voies, un seuil commun, 16 sorties + 1 OR). Le seuil est réglable par l'utilisateur. La carte d'acquisition est une carte de test « USB » configurable qui tire parti de la souplesse de l'interface « USB » des ordinateurs personnels et des progrès des circuits numériques configurables « FPGA » (« Field Programmable Gate Array »). Elle permet de traiter rapidement un grand nombre de signaux et est programmable depuis l'interface d'un ordinateur.

Le schéma de principe est illustré à la figure 1. Le cathéter 21 issu de l'artère de l'animal 2 se prolonge par le micro-tube 28 de la figure 3 arrivant sur un côté du boîtier 6b, puis par le tronçon aplati 4a qui prend le relais à l'intérieur du boîtier 6b, et le sang ressort de l'autre côté de celui-ci. Cette circulation du sang est réalisée grâce à la pompe péristaltique 7. Le volume des microéchantillons prélevés est réglable, ainsi que leur temps de prélèvement. Ces paramètres sont pilotés par l'ordinateur du dispositif de commande 9 de l'installation 1.

L'espacement minimum entre deux prélèvements de microéchantillons est de 1 seconde. Afin de couvrir la dynamique d'une cinétique de radiotraceur dans le sang, les microéchantillons sont prélevés toutes les secondes après l'injection pendant environ 30 secondes à 1 minute, puis ils sont prélevés de façon plus espacée dans le temps, la pente de la courbe étant plus faible pendant cette phase.

Comme expliqué ci-dessus en référence à la figure 3, la connexion entre le cathéter de prélèvement 21 et le micro-tube 28 débouchant dans le boîtier 6b a été réalisée de façon à supprimer tout volume mort qui aurait entraîné un mélange de deux microéchantillons adjacents. Quant aux connexions entre les micro-tubes et le boîtier 6b, elles sont conçues pour éviter toute perte de volume des microéchantillons. En outre, les dimensions des deux micro-tubes externes au boîtier 6b sont telles qu'aucune diffusion entre deux microéchantillons adjacents n'est possible.

Ces microéchantillons sanguins sont ainsi prélevés de façon discrète dans l'espace et dans le temps, et ils progressent sans diffuser jusqu'au système d'échantillonnage 8 où ils sont stockés et avantageusement soumis à l'extraction d'une au moins de leurs phases ou de leurs composants, par exemple mise en oeuvre par centrifugation.

On notera que ce système de prélèvement 3 selon l'invention peut être avantageusement utilisé dans le domaine de la recherche préclinique pour l'imagerie quantitative de nouveaux traceurs, notamment en tomographie par émission de positons (TEP). Dans ce cas, le liquide considéré est du sang, et l'application consiste à mesurer la fonction d'entrée pour de petits animaux comme les rats ou les souris. La faible taille de ces animaux, et donc la faible quantité totale de sang qu'ils possèdent, limitent le volume de chaque microéchantillon à environ 30 µL pour les rats, et à environ 8 µL pour les souris.

## Revendications

1. Dispositif de raccordement fluidique (20) qui est destiné à transférer un fluide, tel que des microéchantillons à prélever ou à injecter, et qui est destiné à être raccordé à un premier conduit (21) par une première ouverture de ce dispositif, lequel comporte une seconde ouverture traversée par un second conduit (28) destiné à communiquer avec le premier conduit pour transférer ce fluide, le dispositif comportant :
- un raccord fluidique femelle (22) qui définit ladite première ouverture et qui comprend une surface interne d'emmanchement (23) se terminant par une extrémité radiale femelle (24) où débouche ce premier conduit, et
- un raccord fluidique mâle (25) qui définit ladite seconde ouverture en étant emmanché dans le raccord femelle via sa surface externe (26) et qui se termine par une extrémité radiale mâle (27) à l'intérieur du raccord femelle,
**caractérisé en ce que** ce second conduit est formé d'un micro-tube souple qui est enfoncé dans le raccord mâle axialement au-delà de ladite extrémité mâle (27), l'extrémité libre de ce second conduit appuyant de manière étanche contre ladite extrémité femelle (24) de sorte à minimiser le volume mort entre le premier conduit et le raccord mâle.

2. Dispositif de raccordement fluidique (20) selon la revendication 1, **caractérisé en ce que** ladite surface interne d'emmanchement (23) du raccord femelle est une surface conique convergeant vers ladite extrémité femelle (24) avec une même conicité que ladite surface externe (26) du raccord mâle qui converge vers ladite extrémité mâle (27).

3. Dispositif de raccordement fluidique (20) selon la revendication 2, **caractérisé en ce que** lesdits raccords mâle (25) et femelle (22) sont tous deux des raccords de type « Luer », tels que définis par la norme ISO 59461 de 1986, ou bien de type « Luer Lock », tels que définis par la norme ISO 594-2 de 1998.

4. Dispositif de raccordement fluidique (20) selon la revendication 3, **caractérisé en ce que** ledit second conduit (28) dépasse de ladite extrémité mâle (27) d'une longueur axiale qui est au moins égale à la distance minimale (dl) séparant lesdites extrémités respectives (24 et 27) des deux raccords (22 et 25) lorsque le raccord mâle est enfoncé en position de connexion dans le raccord femelle (22) conformément à l'une ou à l'autre desdites normes.

5. Dispositif de raccordement fluidique (20) selon une des revendications précédentes, **caractérisé en ce que** ledit second conduit (28) est pourvu, autour de sa paroi cylindrique et à proximité de son extrémité libre, d'un moyen de rigidification (29) apte à le rigidifier à l'intérieur dudit raccord femelle (22).

6. Dispositif de raccordement fluidique (20) selon la revendication 5, **caractérisé en ce que** ledit moyen de rigidification (29) est formé d'une bague en un matériau qui présente une rigidité au moins égale et de préférence supérieure à celle dudit second conduit (28) et qui est apte à être solidarisé avec lui, cette bague étant par exemple à base d'une résine polymérique et étant montée en appui entre ledit second conduit (28) et ladite surface interne d'emmanchement conique (23) dudit raccord femelle (22).

7. Dispositif de raccordement fluidique (20) selon une des revendications précédentes, **caractérisé en ce qu'**il est équipé dudit premier conduit (21) qui est un micro-tube souple adapté pour le prélèvement ou l'injection dudit liquide dans un animal, tel qu'un cathéter souple à implanter dans la veine caudale d'un petit mammifère en vue du prélèvement de microéchantillons sanguins.

8. Système automatisé de prélèvement (3) en continu d'une série de microéchantillons liquides d'un corps (2) contenant un liquide à prélever, tels que des microéchantillons sanguins d'un mammifère de type rat ou souris, le système comportant une succession de conduits (4, 5) qui sont reliés entre eux par une pompe doseuse (7), telle qu'une pompe péristaltique, pour l'aspiration par à-coups d'une quantité déterminée du liquide à acheminer vers un conteneur de stockage (8), cette succession de conduits (4, 5) comportant un premier conduit souple (21) de type cathéter qui est destiné à être implanté dans ce corps et qui est raccordé à un dispositif de raccordement (20) pour le transfert des microéchantillons vers la pompe, **caractérisé en ce que** ce dispositif de raccordement est tel que défini à l'une des revendications précédentes.

9. Système automatisé de prélèvement (3) selon la revendication 8, **caractérisé en ce qu'**il est relié à un dispositif de commande (9) assisté par ordinateur pour la commande de ladite pompe (7).

10. Système automatisé de prélèvement (3) selon la revendication 8 ou 9, **caractérisé en ce que** ladite succession de conduits (4 et 5) présente une section transversale sensiblement constante, de sorte que les microéchantillons s'y succédant circulent chacun sur une longueur axiale qui est supérieure à au moins cinq fois la plus grande dimension transversale intérieure de ces conduits.

11. Système automatisé de prélèvement (3) selon une des revendications 8 à 10, **caractérisé en ce que** les élargissements de section transversale de ladite succession de conduits (4 et 5) sont tous inférieurs ou égaux à 20 % en termes de ratios de surfaces, de telle sorte que les microéchantillons se succédant dans ces conduits soient discrétisés dans l'espace et dans le temps, en particulier pour que ceux dont le volume est inférieur ou égal à 30 µL ne s'y mélangent pratiquement pas entre eux.

12. Système automatisé de prélèvement (3) selon une des revendications 8 à 11, **caractérisé en ce que** ledit micro-tube souple formant ledit second conduit (28) se prolonge par au moins un tronçon (4a) aplati de section transversale oblongue, qui est adapté pour minimiser l'atténuation de particules émises par chaque microéchantillon liquide circulant dans ce tronçon de sorte à optimiser le comptage de ces particules, telles que des électrons ou des positons issus de la désintégration bêta.

13. Système automatisé de prélèvement (3) selon la revendication 12, **caractérisé en ce que** ledit tronçon aplati (4a) présente une section transversale sensiblement rectangulaire dont les grands côtés et/ou les petits côtés sont incurvés selon des courbures symétriques l'une de l'autre, de sorte que ce tronçon présente au moins en partie une face externe sensiblement convexe ou concave.

14. Système automatisé de prélèvement (3) selon la revendication 13, **caractérisé en ce que** ledit tronçon aplati (4a) présente un ratio [hauteur interne (h) / largeur interne (I)] inférieur à 20 % et de préférence compris entre 5 % et 10 %, où la hauteur et la largeur internes représentent respectivement la plus petite et la plus grande des dimensions transversales de ce tronçon, mesurées selon deux directions sensiblement perpendiculaires.

15. Système automatisé de prélèvement (3) selon la revendication 14, **caractérisé en ce que** le ratio surfacique de la section de passage dudit tronçon aplati (4a) sur celle de chaque portion cylindrique adjacente à ce tronçon est inférieur ou égal à 35 %.

16. Système automatisé de prélèvement (3) selon la revendication 15, **caractérisé en ce que** ladite hauteur interne du tronçon aplati (4a) est inférieure à 500 µm et de préférence comprise entre 100 µm et 200 µm, la ou chaque portion cylindrique adjacente présentant un diamètre de l'ordre de 1 mm.

17. Système automatisé de prélèvement (3) selon une des revendications 12 à 16, **caractérisé en ce que** ledit tronçon aplati (4a) présente une paroi d'épaisseur e, exprimée en µm, et de masse volumique d, exprimée en g/cm³, dont le produit e.d est inférieur à 100 et de préférence inférieur à 50, de telle sorte que l'atténuation par ce tronçon de ces particules à compter soit minimisée lorsque ces particules sont des électrons ou des positons.

18. Système automatisé de prélèvement (3) selon la revendication 17, **caractérisé en ce que** ledit tronçon aplati (4a) est à base d'un polyimide, par exemple de dénomination « Kapton ».

19. Système automatisé de prélèvement (3) selon la revendication 18, **caractérisé en ce que** ledit tronçon aplati (4a) présente une paroi d'épaisseur inférieure à 50 µm et de préférence inférieure à 30 µm.

20. Système automatisé de prélèvement (3) selon une des revendications 12 à 19, **caractérisé en ce que** ledit tronçon aplati (4a) est équipé, en regard de ses grandes faces (6aa) et venant en dépassement de ses petites faces (6ab), de deux ensembles de détecteurs (6a) aptes à compter lesdites particules de chaque microéchantillon liquide y circulant.

21. Système automatisé de prélèvement (3) selon la revendication 20, **caractérisé en ce que** lesdits détecteurs (6a) sont agencés contre ou à proximité immédiate desdites grandes faces (6aa).

22. Système automatisé de prélèvement (3) selon une des revendications 8 à 21, **caractérisé en ce que** ladite succession de conduits (4, 5) se termine par un tronçon plus rigide formant une microbuse qui vient en contact avec une paroi réceptrice d'un dispositif aval, le plan de section de l'extrémité de cette microbuse faisant un angle compris entre 5° et 85° avec ladite paroi réceptrice.

23. Système automatisé de prélèvement (3) selon la revendication 20 ou 21, **caractérisé en ce que** les microéchantillons prélevés sont du sang total de mammifère (2) comprenant un plasma et des globules, et **en ce que** ce système comporte, en amont de ladite pompe doseuse (7) qui est pilotée par ordinateur :
- un dispositif de comptage de particules (6) bêta émises par un radiotraceur dilué dans ce sang total, le dispositif de comptage étant également piloté par ordinateur et comprenant à l'intérieur d'un boîtier de mesure (6b) lesdits ensembles de détecteurs (6a), tels que des diodes au silicium, ainsi qu'une carte électronique de traitement et d'interfaçage, et
- un module électronique pour la lecture de ces détecteurs, l'acquisition et le transfert de données.
